# EUROPEAN PATENT SPECIFICATION

(11) **EP 2 997 136 B1**
(45) Date of publication and mention of the grant of the patent: **17.04.2019**
(21) Application number: 14734244.8
(22) Date of filing: 15.05.2014
(51) Int. Cl.: C12N 5/079, G01N 33/50, G01N 33/68

(54) **MODEL OF ALZHEIMER'S DISEASE**
MORBUS-ALZHEIMER-MODELL
MODÈLE DE LA MALADIE D'ALZHEIMER

(30) Priority: 15.05.2013 IN DE14422013
(43) Date of publication of application: 23.03.2016
(73) Proprietor: Council of Scientific & Industrial Research, New Delhi 110 001 (IN)
(72) Inventor: BANDYOPADHYAY, Sanghamitra, Lucknow 226001 (IN); ASHOK, Anushruti, Lucknow 226001 (IN); RAI, Nagendra Kumar, Lucknow 226001 (IN); RAI, Asit, Lucknow 226001 (IN); TRIPATHI, Sachin, Lucknow 226001 (IN)
(74) Representative: Fuchs Patentanwälte Partnerschaft mbB
(86) International application number: PCT/IN2014/000330
(87) International publication number: WO 2014/184809

(56) References cited:
- US-B1- 6 175 057
- A. RAI ET AL: "Characterization of Developmental Neurotoxicity of As, Cd, and Pb Mixture: Synergistic Action of Metal Mixture in Glial and Neuronal Functions", TOXICOLOGICAL SCIENCES, vol. 118, no. 2, 9 September 2010 (2010-09-09), pages 586-601, XP55141369, ISSN: 1096-6080, DOI: 10.1093/toxsci/kfq266 cited in the application
- SUN ET AL: "The strong law under a semiparametric model for truncated and censored data", STATISTICS AND PROBABILITY LETTERS, NORTH-HOLLAND, AMSTERDAM, NL, vol. 76, no. 14, 1 August 2006 (2006-08-01), pages 1550-1558, XP005514154, ISSN: 0167-7152, DOI: 10.1016/J.SPL.2006.03.017
- JAMES A. DUCE ET AL: "Biological metals and Alzheimer's disease: Implications for therapeutics and diagnostics", PROGRESS IN NEUROBIOLOGY, vol. 92, no. 1, 1 September 2010 (2010-09-01), pages 1-18, XP55141546, ISSN: 0301-0082, DOI: 10.1016/j.pneurobio.2010.04.003
- Johannes C.M. Schlachetzki ET AL: "Studying neurodegenerative diseases in culture models", Revista brasileira de psiquiatria, vol. 35, 1 January 2013 (2013-01-01), pages S92-S100, XP055279171, BR ISSN: 1516-4446, DOI: 10.1590/1516-4446-2013-1159
- M Q Xia ET AL: "Expression of the chemokine receptor CXCR3 on neurons and the elevated expression of its ligand IP-10 in reactive astrocytes: in vitro ERK1/2 activation and role in Alzheimer's disease", Journal of neuroimmunology, 1 August 2000 (2000-08-01), pages 227-235, XP055392748, NETHERLANDS Retrieved from the Internet: URL:http://ac.els-cdn.com/S016557280000285 X/1-s2.0-S016557280000285X-main.pdf?_tid=1 cda541a-6d58-11e7-9caa-00000aacb361&acdnat =1500561266_d3048f383aeedda89e7c7c1a77c0be ae

## Description

The present invention relates to the use of a non-transgenic animal wistar rat model of early Alzheimer's disease (AD), characterized by the over-expression of amyloid beta (Aβ) peptides, amyloid precursor protein (APP), intermediate β-secretase (BACE), C-terminal fragment β (CTF-β) and presilin proteins in rat brain cortex and hippocampus. The wistar rat model also relates to vascular damage associated with AD, characterized by alteration in expression of Receptor for advanced glycation end products (RAGE) and P-glycoprotein (Pgp) in rat brain cortex and hippocampus.

The present invention also provides novel neuronal and astrocyte cellular models of AD, characterized by the increase in Aβ peptides, APP, BACE and presilin proteins.

Overall, the invention relates to the generation of an early age non-transgenic rat model, and the use of cellular models of AD through exposure to a mixture of As, Pb and Cd, at a particular dose and time. The generation of the AD parameters is synergistic. These animal and cellular models of AD may be used as convenient tools for studying the etiology of the disease at an early age, and identifying novel compounds targeting the disease.

The AD is characterized by the abnormal processing of APP by BACE to produce Aβ peptides that subsequently aggregate to form the neurodegenerative Aβ plaques. This leads to a significant loss in neuron and synapse, thereby, impairing cognition and inducing dementia. AD also involves impairment in vascular clearance of Aβ contributing to its accumulation in brain. Transgenic animal models for AD have been developed for understanding the *in vivo* mechanisms related to the AD-pathology. The majority of the models created over-express mutant APP, presenilin or tau, and some double or triple transgenics have been generated to overlay pathologies. However, the production of transgenic animals is a time-consuming, laborious and inefficient task, and ethical concerns limit the numbers of animals employed in experiment. The lower species, such as Drosophila, Caenorhabditis elegans, and the Sea lamprey that have been exploited have a brain anatomy much different from humans, making a direct comparison difficult. A non-transgenic method of inducing plaque deposition in a mammal is by infusing Aβ peptide into the brain. A disadvantage of the delivery method is the thinning of the cerebral layers due to chronic implantation of the cannula. Moreover, none of the models render early AD symptoms. Therefore, more and more suitable non-transgenic rodent models are needed for a distinct understanding of the developmental pathophysiology of the disease comprising amyloid as well as vascular pathology.

Culturing cortical and hippocampal cells of brain with Aβ1-42 is the closest prior art for generating cellular models for AD. However, our non-transgenic cellular models for AD are less expensive, quick and efficient. The non-transgenic cellular models are validated with the known AD-targeting drugs and preventive agents.

One of the physiologically relevant environmental factors able to affect the conformation of amyloidogenic proteins is metal ions (Rogers and Lahiri 2004). The heavy metals, such as arsenic (As), cadmium (Cd), and lead (Pb) have received attention as potential neurotoxicological hazards. Studies with single metal exposure have demonstrated that As, Cd, or Pb infiltrates the immature blood-brain barrier (BBB) and accumulates in developing brain leading to neurobehavioral aberrations. We have reported that a combination of the three metals, at human exposure relevant doses, compromised the BBB leading to behavioral dysfunction during early rat brain development (Rai et al. 2010).

We, therefore, hypothesized that the mixture of As, Cd and Pb, at the human relevant doses, may induce AD-like symptoms in developing rats and promotes vascular damage. The mixture may provoke the synergistic generation of AD-marker genes and proteins, at an early age. Accordingly, the present invention provides the use of a novel non-transgenic animal wistar rat model of early Alzheimer's disease and neuronal and astrocyte cellular model of AD, showing a synergistic increase in amyloidogenicity due to exposure to a heavy metal mixture of As, Cd and Pb.

### NON-TRANSGENIC EARLY WISTAR RAT MODEL:

An embodiment of the present invention is to provide a synergistic and dose-dependent induction of Aβ-40, -42 and APP in rat brain cortex and hippocampus, at an early age, by orally feeding developing wistar rats with a heavy metal mixture in water. The control animals are fed with water devoid of the metals (vehicle). The heavy metals are As, Cd and Pb, fed in the form of NaAsO₂, CdCl₂ and Pb(C₂H₃O₂)₂, respectively, in Milli Q water. The NaAsO₂, and CdCl₂ readily dissolve in water, while Pb(C₂H₃O₂)₂ is dissolved by adding 0.043 % acetic acid in water. The treatment with the metals starts at gestation day-5 (G-05), continues through gestation, and until post-natal day- -90 (P-90). The increase in Aβ-40 peptide, compared to age-matched controls, ranges from 1.3 ± 0.1063-fold (lower dose) to 1.5±0.1052-fold (higher dose) in cortex, and from 1.5 ± 0.1077-fold (lower dose) to 1.8 ± 0.1909-fold (higher dose) in hippocampus. The increase in Aβ-42 peptide ranges from 1.7 ± 0.1028-fold (lower dose) to 2.4 ± 0.1098-fold (higher dose) in cortex, and from 1.7 ± 0.0875-fold (lower dose) to 3.0 ± 0.1452-fold (higher dose) in hippocampus. The increase in APP compared to age-matched controls ranges from 1.5 ± 0.0959-fold (lower dose) to 2.2 ± 0.1032-fold (higher dose) in cortex, and from 1.3 ± 0.0461-fold (lower dose) to 3.0 ± 0.1498-fold (higher dose) in hippocampus. *Another embodiment of the present invention is to provide a time-dependent induction of Aβ_{1- 40}, Aβ₁₋₄₂, and APP in rat brain cortex and hippocampus, at an early age, by orally feeding developing wistar rats with a heavy metal mixture in water. The control animals are fed with water devoid of the metals (vehicle). The heavy metals are As, Cd and Pb, fed in the form of NaAsO₂, CdCl₂ and Pb(C₂H₃O₂)₂, respectively, in Milli Q water. The NaAsO₂, and CdCl₂ readily dissolve in water, while Pb(C₂H₃O₂)₂ is dissolved by adding 0.043 % acetic acid in water. The treatment with the metals starts at gestation day-5 (G-05), continues through gestation until post-natal day-24 (P-24), -60 (P-60) or -90 (P-90).*

Another embodiment of the present invention is to provide a synergistic induction of BACE enzymatic activity in wistar rat cortex and hippocampus, at an early age, by orally feeding wistar rats from G-05 until P-60 or P-90 with a mixture of As, Cd and Pb, in the form of NaAsO₂ CdCl₂ and Pb(C₂H₃CO₂)₂, in Milli Q water. The increase in BACE compared to age-matched controls ranges from 1.3 ± 0.1450-fold (60 day) to 2.0 ± 0.2679-fold (90 day) in cortex, and from 1.26 ± 0.0510-fold (60 day) to 1.5 ± 0.2733-fold (90 day) in hippocampus. Another embodiment of the present invention is to provide an induction of C-terminal fragment (APP-CTF-β) and presenilin in wistar rat brain, at an early age, by orally feeding wistar rats from G-05 until P-90 with a mixture of As, Cd and Pb in the form of NaAsO₂, CdCl₂ and Pb(C₂H₃CO₂)₂, in Milli Q water. The increase in APP-CTF-β is around 1.3 ± 0.0908-fold and 1.5 ± 0.1347-fold in cortex and hippocampus respectively. The increase in presenilin is around 1.7 ± 0.1635-fold and 1.6 ± 0.1066-fold in cortex and hippocampus respectively.

Another embodiment of the present disclosure is to provide an induction of Aβ-mediated apoptosis in the brain by orally feeding wistar rats from G-05 until P-60 or P-90 with a mixture of As, Cd and Pb, in the form of NaAsO₂, CdCl₂ and Pb(C₂H₃CO₂)₂, in Milli Q water. The increase in Aβ-mediated-apoptosis is 11 ± 2.1369-fold (60 day) to 16 ± 1.3558-fold (90 day) in rat brain.

vi. Another embodiment of the present disclosure is to provide an induction of RAGE, and suppression of p-GP in the brain by orally feeding wistar rats from G-05 until P-90 with a mixture of As, Cd and Pb, in the form of NaAsO₂ CdCl₂ and Pb(C₂H₃O₂)₂ in Milli Q water. Another embodiment of the present disclosure is to provide an induction of cognition-loss, at an early age, by orally feeding wistar rats from G-05 until P-90 with a mixture of As, Cd and Pb, in the form of NaAsO₂, CdCl₂ and Pb(C₂H₃CO₂)₂, in Milli Q water. The loss in cognition compared to vehicle is around 35%.

Preferably, an induction of the interleukin-1 (IL-1α), IL-1β and interleukin-1 receptor (IL-1R1) and the inflammatory marker, Iba-1, that provide a casual link between the prominent reactive gliosis and neuritic plaque formation in the wistar rat cortex and hippocampus is provided, using the heavy metal mixture of As, Cd and Pb. The inflammation may be induced by orally feeding wistar rats from G-05 until P-60 or P-90 with a mixture of As, Cd and Pb, in the form of NaAsO₂, CdCl₂ and Pb(C₂H₃O₂)₂, in Milli Q water. The increase in IL-1α, IL-1β, and IL-1R1 ranges from 1.6 ± 0.1794-fold (60 day), 1.3 ± 0.1194-fold (60 day), and 1.3 ± 0.1104-fold (60 day) to 1.7 ± 0.1037-fold (90 day), 1.8 ± 0.1901-fold (90 day) and 1.5 ± 0.0769-fold (90 day) respectively. Therefore, this model could be used for identifying drugs that activate inflammation, via IL-1α, IL-1β andIL-1R1. It could also be helpful in identifying inflammatory mechanism in wistar rat cortex and hippocampus, as such, and pertaining to AD specifically. Another embodiment of the present invention is to provide a non-transgenic wistar rat model for AD that may serve as a screening tool for compounds targeted to the disease. The known AD-targeting drugs, memantine (10mg/kg body weight) and donepezil (conc. 1.5 mg/kg body weight), suppress Aβ₁₋₄₂, Aβ₁₋₄₀ and APP in this non-transgenic wistar rat model of AD. Omega-3 fatty acid (mixture of eicosapentaenoic acid, 90mg/Kg, and docosahexaenoic acid, 60mg/Kg) and α-tocopherol (100mg/Kg body weight) suppress Aβ₁₋₄₂ and APP in this non-transgenic wistar rat model of AD.

Another embodiment of the present invention is to provide an induction of Aβ-42 and APP in rat brain cortex and hippocampus, at an early age (age is different from embodiment i), by orally feeding wistar rats from P-90 until P-120 with a mixture of As, Cd and Pb, in the form of NaAsO₂, CdCl₂ and Pb(C₂H₃O₂)₂ in Milli Q water. The increase in the APP and Aβ₁₋₄₂ levels are around 1.4 ± 0.0808-fold and 1.5 ± 0.2274-fold respectively at P-120.

### NEURONAL AND ASTROCYTE CELLULAR MODEL OF AD:

An embodiment of the present invention is to provide an induction of Aβ-42, APP and BACE in rat neuronal and astrocyte cultures, treated with a mixture of As, Cd and Pb, in the form of NaAsO₂, CdCl₂ and Pb(C₂H₃O₂)₂ respectively. The effect is synergistic. The NaAsO₂, and CdCl₂ readily dissolve in water, while Pb(C₂H₃O₂)₂ is dissolved by adding 0.043 % acetic acid in water. The 80% confluent astrocytes and neurons are treated with the heavy metal mixture and incubated for 16 hr. The increase in the levels of Aβ, APP and BACE is 2.3 ± 0.2232-fold, 2.0 ± 0.1949-fold and 1.5 ± 0.1917-fold respectively in neurons, and 2.2 ± 0.2015-fold, 2.3 ± 0.2247-fold and 2.0 ± 0.2682-fold respectively in astrocytes.

The present invention provides a non-transgenic wistar neuronal and astrocyte models for AD that serves as screening tools for compounds targeted to the disease. Memantine (conc. 5 µM and Brand name is Admenta, Sun Pharma Sikkim) and donepezil (conc. 10 µM is and Brand name: Aricep 10, Eisai Co. Ltd) suppressed the levels of Aβ and APP in the cellular model of AD. The omega 3 fatty acid (docosahexaenoic acid, 10 µM, from Sigma-Aldrich), known to reduce the risk of AD, suppressed the levels of Aβ and APP in the cellular model of AD. Vitamin E (conc. 10 µM, Sigma-Aldrich) known to be protective against AD, suppressed the levels of Aβ and APP in the cellular model of AD.

Another embodiment of the present invention is to provide a synergistic induction of Aβ-mediated apoptosis in the cultured neurons and astrocytes, treated with a mixture of As, Cd and Pb, in the form of NaAsO₂, CdCl₂ and Pb(C₂H₃O₂)₂ respectively in autoclaved MilliQ water. The 80% confluent astrocytes and neurons are treated with the heavy metals and incubated for 16 hr.

Another embodiment of the present disclosure is to provide an induction of the inflammatory cytokines, IL-1α and IL-1β, and a rise in the IL-1R1 in the rat brain cells treated with a mixture of As, Cd and Pb, in the form of NaAsO₂, CdCl₂ and Pb(C₂H₃O₂)₂ respectively. The 80% confluent brain cells are treated with the heavy metal mixture and incubated for 16 hr. Therefore, this cellular model could be used for identifying drugs that suppress inflammation, via IL-1α and IL-1β with the up-regulation of IL-1R1. It could also help in identifying inflammatory mechanism in wistar rat cortex and hippocampus, as such, and pertaining to AD specifically. The increase in IL-1α and IL-1β in the brain cells is around 1.7 ± 0.0600-fold and 1.5 ± 0.1021-fold respectively. The rise in IL1-R1 in neurons and astrocytes is around 2.3 ± 0.3241-fold and 2.9 ± 0.0792-fold respectively.

These novel non-transgenic animal and cellular models of AD can be used (i) for investigating early-age etiology and mechanistic modulations in AD, and (ii) for screening and identifying novel compounds targeting the pathological hallmarks of AD.

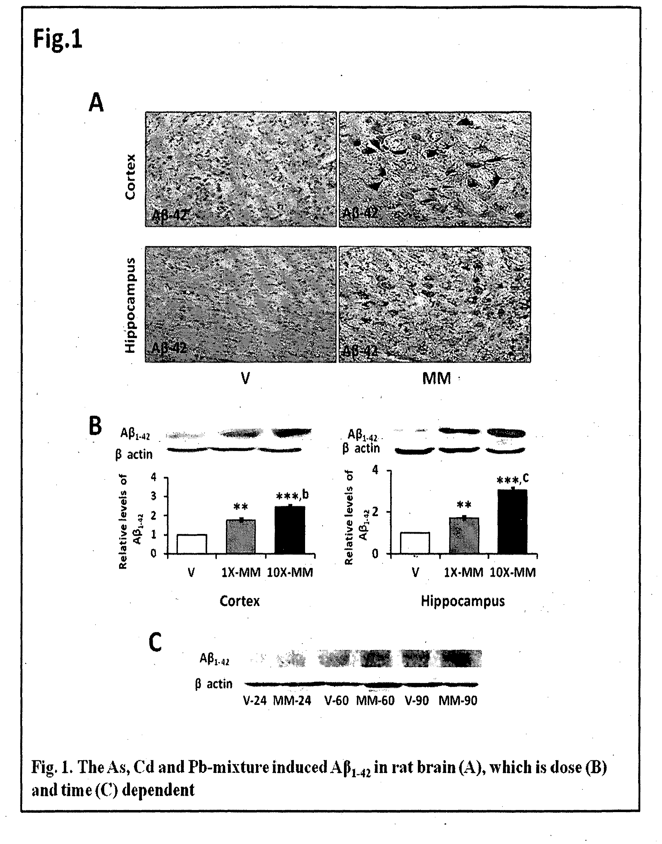

**Figure 1****: The As, Cd and Pb-mixture induced Aβ1-42 in rat brain (A), which is dose (B) and time (C) dependent :(A)** 5-µm transverse sections were made from the cortex of vehicle (V) and metal mixture (MM, 10X)-treated 90 day wistar rats, immunolabelled with Aβ1-42 through DAB staining and photographed with a light microscope (40X magnification).
   **(B)** The cortical and hippocampal tissues from vehicle (V) and metal mixture (MM, 1X and 10X, refer to Table 1)-treated 90 day wistar rats were immunoblotted for Aβ1-42 and β-actin. Representative western blot and densitometry of Aβ1-42 normalized with β-actin.Data represent means ± SE of three pups from three different litters. ****P*<0.001, and ***P*<0.01 indicate significant changes in the metal mixture treated rats compared to V. ^{c}*P*<0.001 and ^{b}*P*<0.01 indicate significant difference compared to 1X-MM. **(C)** The hippocampal tissues from vehicle (V) and metal mixture (MM)-treated postnatal 24, 60 and 90 day wistar rats were immunoblotted for Aβ1-42 and β-actin. Representative western blot of Aβ1-42 normalized with β-actin.

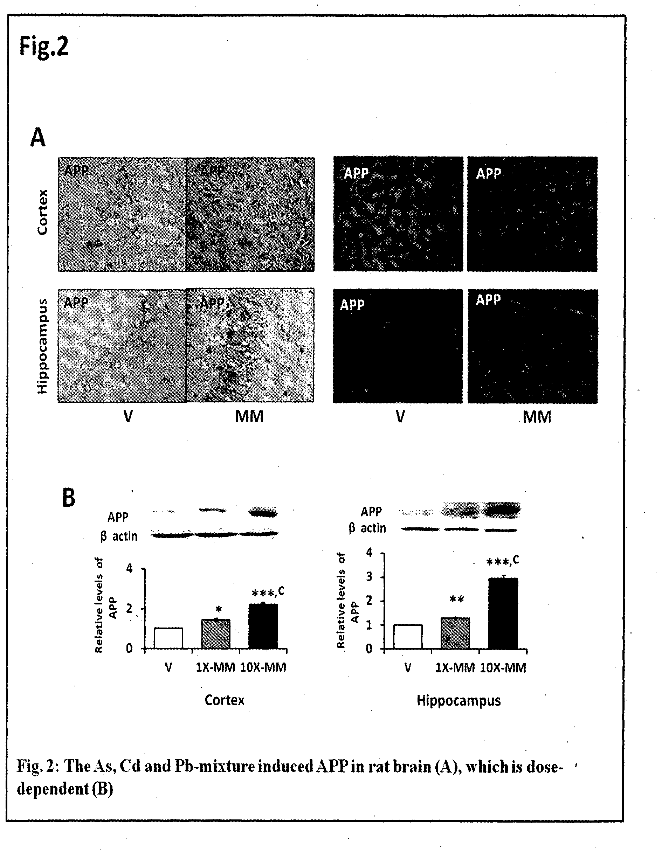

**Figure 2****: The As, Cd and Pb-mixture induced APP in rat brain (A), which is dose-dependent (B)- (A)** Representative photomicrographs of the cortical sections from the vehicle (V) and MM treated 90 day rats that were labelled for APP through DAB staining **(LHS)** and immunofluorescence **(RHS)**.
   **(B)** Cortical and hippocampal tissues from vehicle (V) and metal mixture (MM, 1X and 10X, refer to Table 1)-treated wistar rats were immunoblotted for APP and β-actin. Representative western blot and densitometry of APP normalized with β-actin.Data represent means ± SE of three pups from three different litters. ****P*<0.001, ***P*<0.01 and **P*<0.05 indicate significant changes in the metal mixture treated rats compared to V. ^{c}*P*<0.001 indicates significant difference compared to 1X-MM.

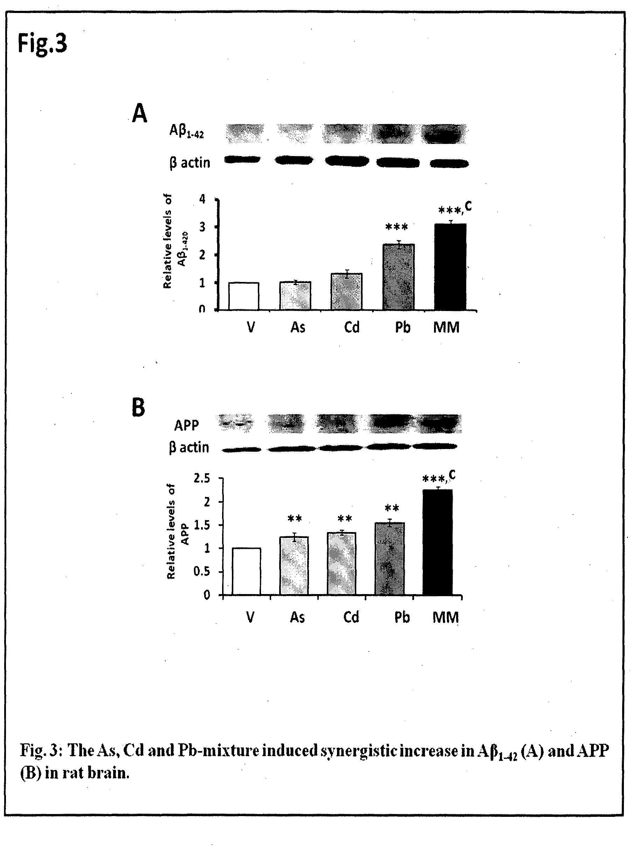

**Figure 3****: The As, Cd and Pb-mixture induced synergistic increase in Aβ1-42 (A) and APP (B) in rat brain** - Representative western-blot and densitometry of Aβ1-42 **(A)** and APP **(B)** normalized with β-actin in cortical tissues in individual metal (As, Cd or Pb) and MM treated rats. Data represent means ± SE of three pups from three different litters. ****P*<0.001, ***P*<0.01 indicate significant changes in the treated rats compared to V. ^{c}*P*<0.001 indicates significant difference compared to individual metals.

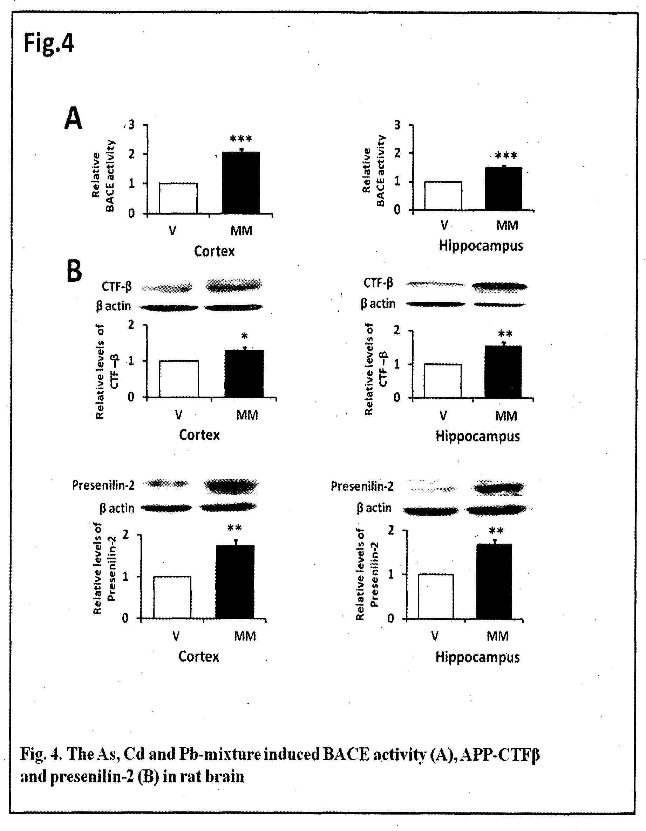

**Figure 4****: The As, Cd and Pb-mixture induced BACE activity (A), APP-CTFβ and presenilin-2 (B) in rat brain- (A)** Representative graph showing fold change in the activity of beta-secretase (BACE) in cortical and hippocampal tissues. ****P*<0.001 indicates significant changes in the metal mixture treated rats compared to V.
   **(B)** Representative western-blot and densitometry of C-terminal fragment of APP (APP-CTF-β) and presenilin-2 normalized with β-actin in cortical and hippocampal tissues. ***P*<0.01 and **P*<0.05 indicate significant changes in the metal mixture treated rats compared to V.

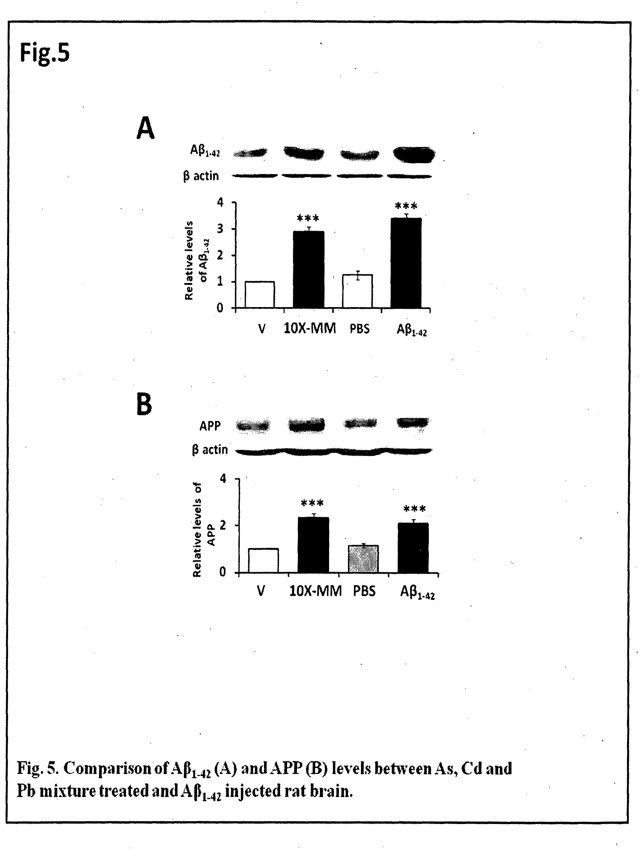

**Figure 5****: Comparison of Aβ1-42 (A) and APP (B) levels of As, Cd and Pb mixture treated and Aβ1-42 injected rat brain-** Representative western-blot and densitometry of Aβ1-42 (A) and APP **(B)** normalized with β-actin in hippocampal tissues in 10X-MM-treated or Aβ1-42-injected rats. ****P*<0.001 indicates significant changes in the metal mixture-treated or Aβ1-42-injected rats compared to respective vehicle.

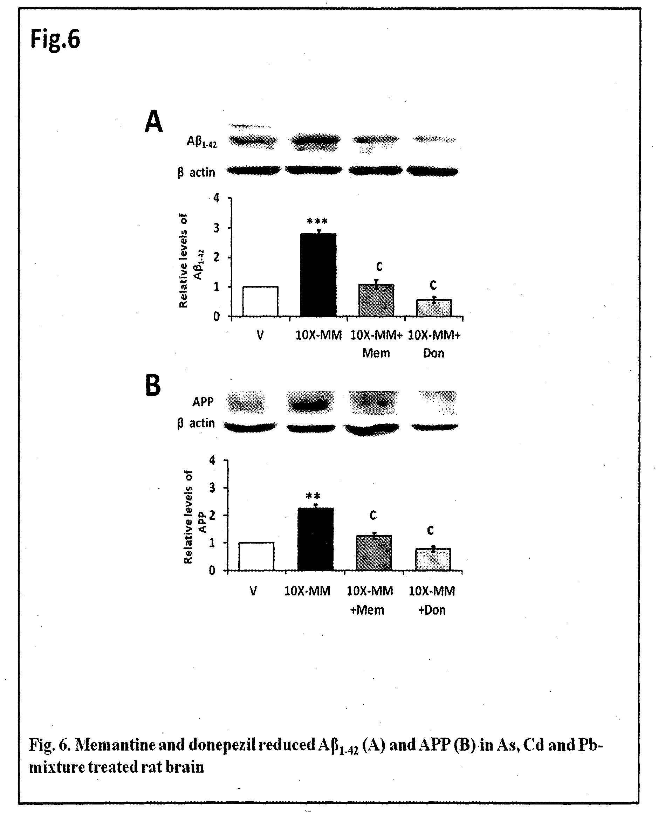

**Figure 6****: Memantine and donepezil reduced Aβ1-42 (A) and APP (B) in As, Cd and Pb-mixture treated rat brain** - The hippocampal tissues from vehicle (V), metal mixture (10X-MM), and metal mixture with memantine (Mem) or donepezil (Don)-treated rats were immunoblotted for Aβ1-42, APP and β-actin. Representative western blot and densitometry of Aβ1-42 **(A)** and APP **(B)** normalized with β-actin. ****P*<0.001 and ***P*<0.01 indicate significant changes in the metal mixture treated rats compared to vehicle. ^{c}*P*<0.001 indicates significant changes in the10X- MM and memantine or donepezil treated rats compared to 10X-MM.

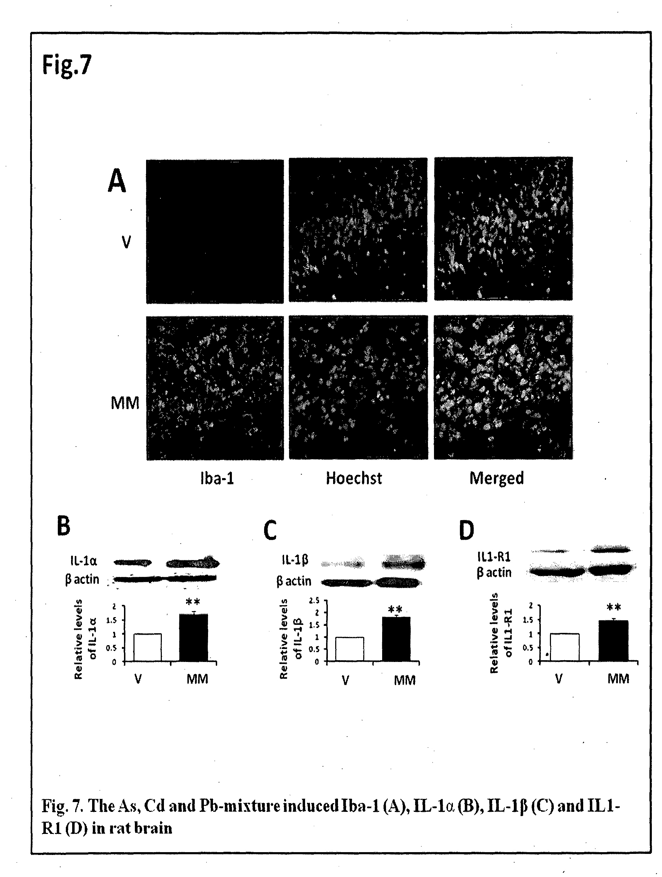

**Figure 7****: The As, Cd and Pb-mixture induced Iba-1 (A), IL-1α (B), IL-1β (C) and IL1-R1 (D) in rat brain - (A)** Representative photomicrograph of Iba-1-positive cells (green-fluorescence), nucleus (blue-fluorescence), and the two merged in the same field.
   The vehicle (V) and metal mixture (MM)-treated brain tissues were immunoblotted for IL-1α, IL-1β, IL-1R1 and β-actin. **(B)** Representative western blot and densitometry of IL-1α normalized with β-actin in rat brain hippocampus. **(C)** Representative western blot and densitometry of lL-1β normalized with β-actin in rat brain hippocampus. **(D)** Representative western blot and densitometry of IL-1R1 normalized with β-actin in rat brain hippocampus. ***P*<0.01 indicates significant changes in the metal mixture treated rats compared to vehicle.

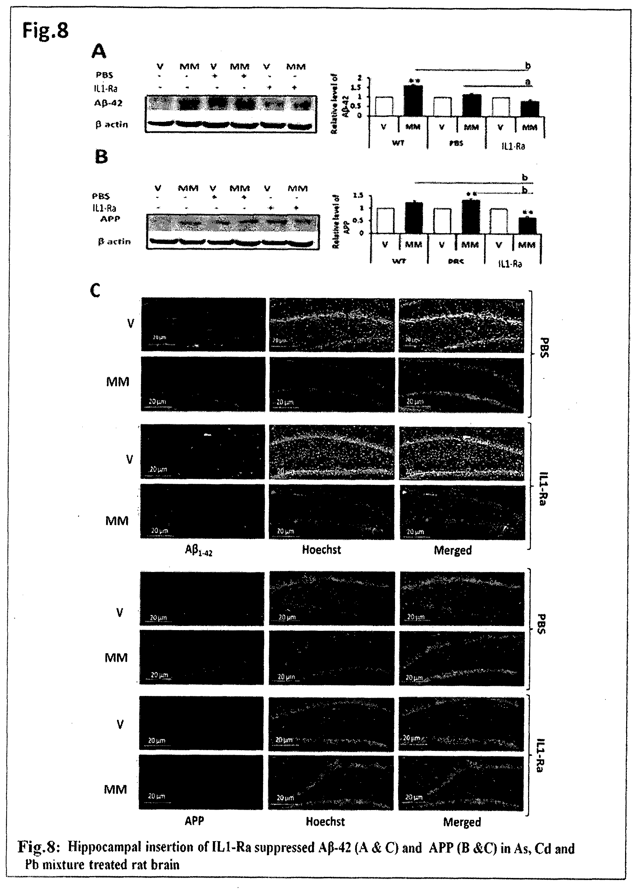

**Figure 8****: Hippocampal insertion of IL1-Ra suppressed Aβ1-42 (A and C) and APP (B and C) in As, Cd and Pb-mixture treated rat brain- Vehicle (V) and MM treated rats were** injected with sterile PBS and IL1-Ra, and western blotting with brain tissues was performed for Aβ1-42, APP and β-actin. Representative western-blot and densitometry of Aβ1-42 **(A)** and APP **(B)** normalized with β-actin. ***P* <0.01 indicates significant changes in the metal mixture treated rats compared to vehicle, ^{a}*P*<0.05 and ^{b}*P*<0.01 indicate significant changes in the IL1-Ra treated rats compared to MM. **(C)** Representative photomicrograph showing Aβ1-42 or APP (red-fluorescence), nucleus (blue-fluorescence), and the two merged in the same field in hippocampus.

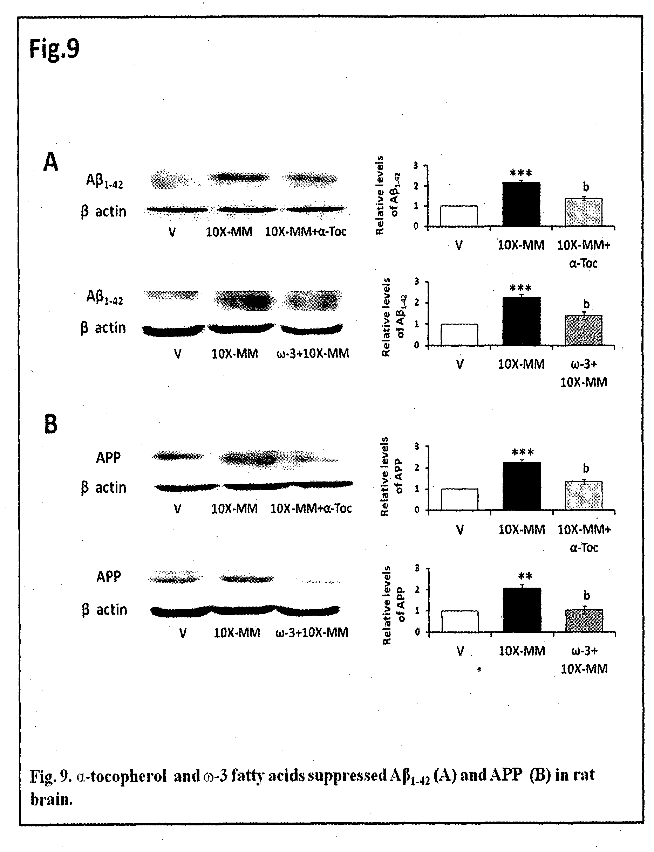

**Figure 9****: α-tocopherol and ω-3 fatty acids suppressed Aβ1-42 (A) and APP (B) in rat brain- (A)** Representative western-blot and densitometry of Aβ1-42 **(A)** or APP **(B)** normalized with β-actin in hippocampal tissues of 10X-MM, 10X-MM and α-tocopherol (α-toc) or 10X-MM and ω-3-fatty acid (ω-3)-treated rats. ****P*<0.001 and ***P*<0.01 indicate significant changes in the 10X-MM-treated rats compared to V. ^{b}*P*<0.01 indicates significant changes in the α-toc or ω-3 treated rats compared to 10X-MM.

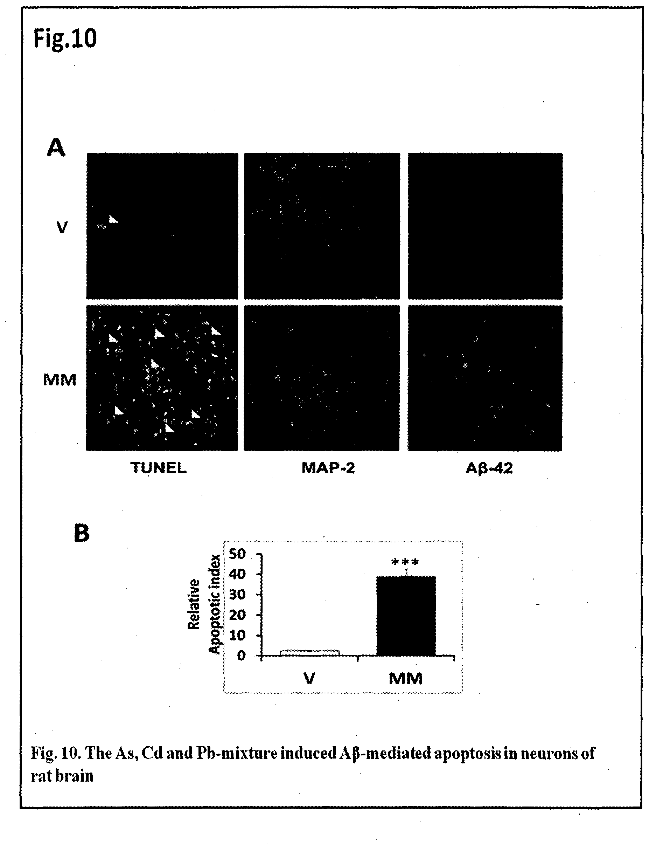

**Figure 10****: The As, Cd and Pb-mixture induced Aβ-mediated apoptosis in neurons of rat brain- (A).** Representative photomicrograph of apoptotic TUNEL (green- fluorescence), co-labelled with neuron-expressing, MAP-2 (red-fluorescence), and serially immunolabelled with Aβ1-42 (red-fluorescence) in the same field in cerebral cortex. **(B).** Representative graph showing relative apoptotic index of MM-treated rats with respective vehicle. ****P*<0.001 indicates significant changes in the metal mixture treated rats compared to vehicle.

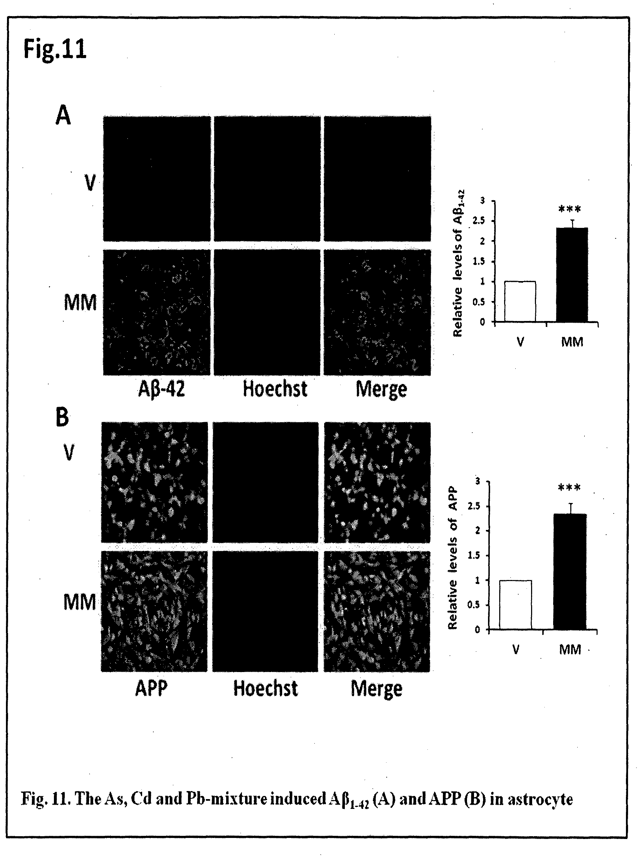

**Figure 11****: The As, Cd and Pb-mixture induced Aβ1-42 (A) and APP (B) in astrocytes-** The vehicle (V) and metal mixture (MM, refer to Table 3 for conc.)-treated astrocytes were immunostained for Aβ1-42 or APP and hoechst. **(A)** Representative photomicrograph of Aβ1-42 immunofluorescence and hoechst, and the two merged together in the same field. **(B)** Representative photomicrograph of APP immunofluorescence and hoechst, and the two merged together in the same field. The bar diagram indicates the number of Aβ1-42 or APP immunoreactive cells normalized with hoechst-+ve nuclei. ****P*<0.001 indicates significant changes in the metal mixture treated astrocytes compared to vehicle.

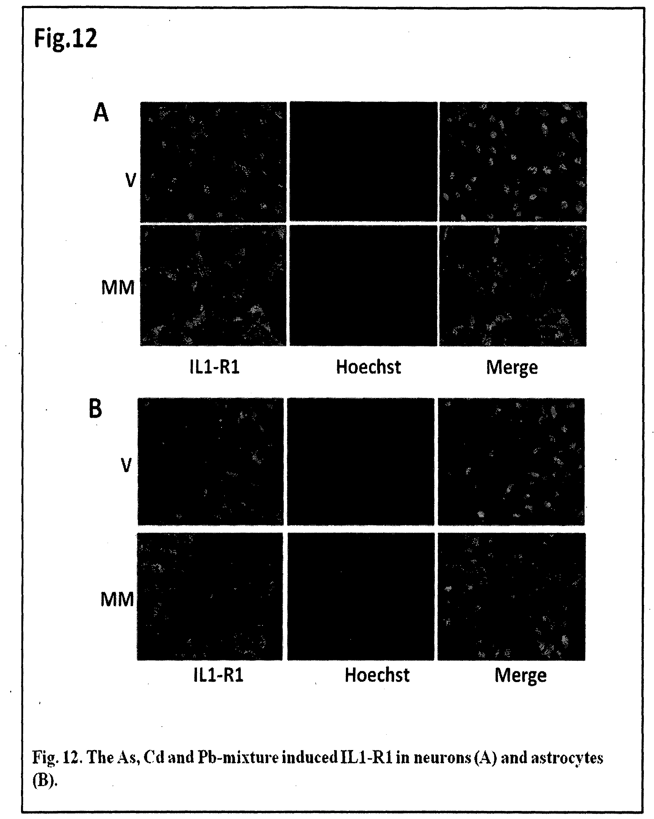

**Figure 12****: The As, Cd and Pb-mixture induced IL1-R1 in neurons (A) and astrocytes (B)-** The vehicle (V) and metal mixture (MM, refer to Table 2 and 3 for conc.)-treated neurons and astrocytes were immunostained for IL1-R1 and hoechst. **(A)** Representative photomicrograph of IL1-R1 (red fluorescence) and hoechst, and the two merged together in the same field in neuronal cells. **(B)** Representative photomicrograph of IL1-R1 (red fluorescence) and hoechst, and the two merged together in the same field in astrocytes.

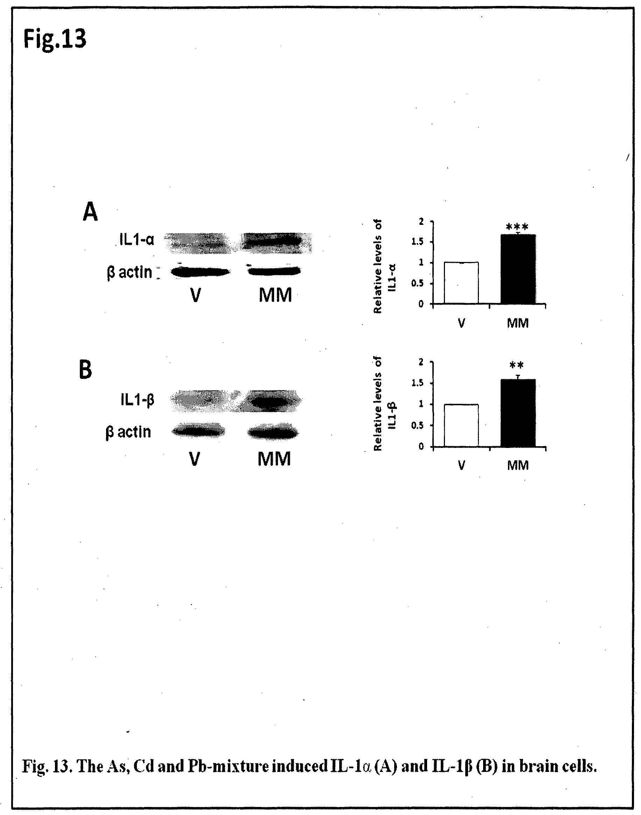

**Figure 13****: The As, Cd and Pb-mixture induced IL-1α (A) and IL-1β (B) in brain cells** - Representative western blot and densitometric analysis of IL1-α **(A)** and IL1-β **(B)** relative to β actin in mixed brain culture treated with MM. ****P*<0.001 and ***P*<0.01 indicate significant changes in the metal mixture treated brain cells compared to vehicle.

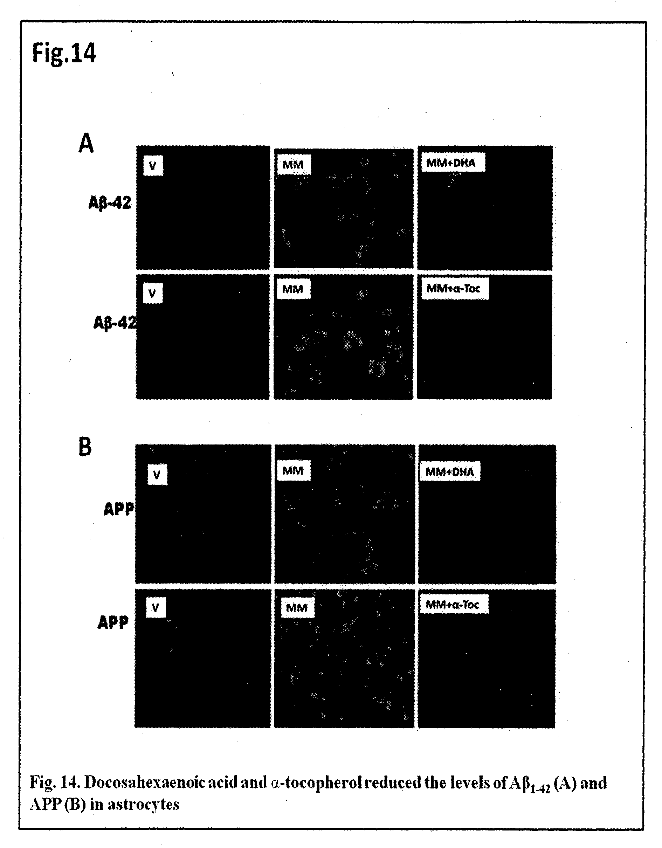

**Figure 14****: Docosahexaenoic acid and α-tocopherol reduced the levels of Aβ1-42 (A) and APP (B) in astrocytes-** The vehicle (V)- and metal mixture (MM), docosahexaenoic aid (DHA) or α-tocopherol-treated astrocytes were immunostained for Aβ1-42 or APP. **(A)** Representative photomicrograph of Aβ1-42 immunofluorescence. **(B)** Representative photomicrograph of APP immunofluorescence.

The main objective of the present invention is to provide a new non transgenic animal wistar rat model of early Alzheimer's disease AD, and to offer new non-transgenic neuronal and astrocyte cellular models of AD.

The aims are to make available the novel animal and cellular models bearing the signs of AD pathology, vascular damage, neuro-inflammation, and neuronal apoptosis associated with AD, for understanding the etiology and mechanisms underlying this disease at an early age. The models will also serve as screening tools for identifying novel drugs that could target AD.

A heavy metal mixture of As, Cd and Pb, in the present invention, is utilized for the development of an early and potential model for AD. The detailed procedures for the development of the model will be discussed, and subsequently the properties matching that of AD. The methods in the following examples should, however, not be construed to limit the scope of invention.

Accordingly, the main embodiment of the present invention provides a non-transgenic animal wistar rat model of early Alzheimer's disease characterized in having over-expression of Aβ and APP proteins in the brain by exposing to a mixture of heavy metals arsenic, cadmium and lead at 0.38 mg/Kg, 0.098 mg/Kg, 0.220 mg/Kg to ten times concentration of each respectively, in water.

Another embodiment of the present invention provides a method for preparing a non-transgenic animal wistar rat model of early Alzheimer's disease as claimed in claim 1 comprising of:
(a) providing a wistar rat;
(b) providing a heavy metal mixture of arsenic, cadmium and lead as claimed in claim 1; and
(c) orally feeding the metal mix as obtained in step (b) to the rat to obtain the non transgenic wistar rat model of Alzheimer's disease.

Another embodiment of the present invention provides a method described as herein in the present invention wherein the oral feeding of heavy metal mixture is started at gestation day - 05 (in pregnant and lactating dams) and continued in the off-springs until early adulthood, i.e. postnatal 60 - 90.

Another embodiment of the present invention provides a method described as herein in the present invention wherein the oral feeding of heavy metal mixture at 3.8 mg/Kg, 0.98 mg/Kg, 2.20 mg/Kg is started at gestation day - 05 (in pregnant and lactating dams) and continued in the off-springs until weaning, i.e. postnatal 24.

Another embodiment of the present invention provides a method described as herein in the present invention wherein the oral feeding of heavy metal mixture at 3.8 mg/Kg, 0.98 mg/Kg, 2.20 mg/Kg is started at postnatal day - 90 and continued until adulthood, i.e. postnatal-120. Another embodiment of the present invention provides a non-transgenic wistar rat model of early Alzheimer's disease characterized in having over-expression of BACE, CTFβ and presenilin in the brain by exposing to a mixture of heavy metals arsenic, cadmium and lead at 3.8 mg/Kg, 0.98 mg/Kg, 2.20 mg/Kg respectively in water.

Another embodiment of the present invention provides a method described as herein in the present invention wherein the heavy metal mixture composition consists of arsenic, cadmium and lead in water.

Another embodiment of the present invention provides a method described as herein in the present invention wherein the heavy metal mixture composition comprising of NaAsO₂ + CdCl₂ + Pb(C₂H₃O₂)₂ at 0.38 mg/Kg, 0.098 mg/Kg, 0.220 mg/Kg to ten times concentration of each respectively, in water.

Another embodiment of the present invention provides use of the rat model as described herein in the present invention for screening anti-Alzheimer's drug.

Another embodiment of the present invention provides use of the rat model as described herein in the present invention for developing anti- Alzheimer's therapies.

Another embodiment of the present invention provides a use of the rat model as described herein in the present invention for detecting early age Alzheimer's disease.

Another embodiment of the present invention provides use of the rat model as described herein in the present invention for screening drugs and developing therapies targeted to BACE, CTFβ and presenilin.

Another embodiment of the present invention provides a composition for the induction of early Alzheimer's disease in a subject comprising of NaAsO₂ + CdCl₂ + Pb(C₂H₃O₂)₂ at 0.38 mg/Kg, 0.098 mg/Kg, 0.220 mg/Kg to ten times concentration of each respectively, in water. Another embodiment of the present invention provides a composition described as herein in the present invention wherein the subject is a non-transgenic wistar rat.

Another embodiment of the present invention provides a neuronal model of Alzheimer's disease characterized in having over-expression of Aβ, APP and BACE proteins by exposing to a mixture of heavy metals arsenic, cadmium and lead.

Another embodiment of the present invention provides for a method of preparing the neuronal model of Alzheimer' disease as described herein in the present invention comprising steps:
(a) isolating neurons from embryonic day 14-16 wistar rats;
(b) preparing mixture of arsenic, cadmium and lead at 5 µM, 1 µM, and 10µM respectively; and
(c) treating the 80% confluent neurons obtained in step a with the mixture obtained in step b to obtain the neuronal model of Alzheimer' disease.

Another embodiment of the present invention provides an astrocyte model of Alzheimer's disease characterized in having over-expression of Aβ, APP and BACE proteins by exposing to a mixture of heavy metals arsenic, cadmium and lead.

Another embodiment of the present invention provides for a method of preparing the astrocyte model of Alzheimer' disease described as herein in the present invention said method comprising steps:
(a) isolating astrocytes from postnatal day-1 wistar rats;
(b) preparing mixture of arsenic, cadmium and lead at 6 µM, 2 µM, and 50 µM respectively; and
(c) treating the 80% confluent astrocytes obtained in step a with the mixture obtained in step b to obtain the astrocyte model of Alzheimer' disease.

Another embodiment of the present invention provides a composition for preparing neuronal and astrocyte model of Alzheimer's disease comprising of NaAsO₂ + CdCl₂ + Pb(C₂H₃CO₂)₂ in water.

Another embodiment of the present invention provides for a use of the neuronal model as described herein in the present invention for screening anti- Alzheimer drugs.

Another embodiment of the present invention provides for a use of the neuronal model as described herein in the present invention for developing anti-Alzheimer therapies.

Another embodiment of the present invention provides for a use of the astrocyte model as described in the herein in the present invention for screening anti- Alzheimer drugs.

Another embodiment of the present invention provides for use of the astrocyte model as described herein in the present invention for developing anti-Alzheimer therapies.

### Procurement details:

Sodium arsenite, lead acetate, cadmium chloride, protease inhibitor cocktail, Hoechst 33258 stain, poly-L-lysine, mammalian tissue protein extraction reagent, , rabbit monoclonal antibody to Aβ-42, mouse monoclonal antibodies to β-actin and peroxidase conjugated secondary antibodies, were purchased from Sigma Chemical Co. (St. Louis, MO). The sample loading buffer for western blotting, protein markers and Alexa fluor secondary antibodies were purchased from Invitrogen (Carlsbad, CA). The supersignal west femto maximum sensitivity substrate for western blotting was purchased from PIERCE Biotechnology (Rockford, IL). Rabbit monoclonal antibody to APP and Aβ-42 were purchased from Abcam (Cambridge, MA). Beta secretase enzyme activity kit was purchased from Abcam (Cambridge, MA). Interleukin-1 alpha, and interleukin-lbeta were purchased from R&D systems (Minneapolis, Mn). Diaminobenzidin tetrahydrochloride (DAB) substrate kit, vectashield medium and Elite ABC kit were purchased from Vector Laboratorie (Burlingame, CA). The terminal deoxynucleotide-transferase (TdT)- Mediated dUTP nick end labelling (TUNEL) kit was purchased from Roche (Indianapolis, IN). ELISA kits for Aβ-40 were purchased from IBL, Japan. Memantine (Brand name, Admenta) and donepezil (Brand name, Aricep 10) were from Sun Pharma, Sikkim and Eisai Co. Ltd, Tokyo, Japan respectively. Omega (ω) 3 fatty acid (Brand name: MAX-EPA) and α-tocopherol were from Merck, India and Sigma Chemical Co. (St. Louis, MO) respectively. DMEM/F-12, Neurobasal media, antibiotics, fetal bovine serum (FBS) and Trypsin-EDTA were from Gibco BRL, USA. Rat recombinant IL-1 receptor antagonist (IL1-Ra) was purchased from R&D systems (Minneapolis, Mn). Rat recombinant Aβ1-42 peptide was purchased from Tocris biosciences (Bristol, United Kingdom).

### Antibody details

Primary* and secondary** antibodies used for western blotting (WB), immunohistochemistry (IHC) and immunocytochemistry (ICC)

| **Antibody** | **Cat. No.** | **Source and location** | **Species** | **Dilution** |
|---|---|---|---|---|
| **APP *** | **ab15272** | **Abcam, Cambridge, MA** | **Rabbit** | **1:1000 (WB), 1:100 (IHC, ICC)** |
| **Aβ₁₋₄₂ *** | **ab10148** | **Abcam, Cambridge, MA** | **Rabbit** | **1:1000 (WB), 1:100 (IHC, ICC)** |
| **Iba-1*** | **ab15690** | **Abcam, Cambridge, MA** | **Mouse** | **1:100 (IHC, ICC)** |
| **CTF-β*** | **A8717** | **Sigma Chemical Co., St. Louis, MO** | **Rabbit** | **1:1000 (WB)** |
| **Presenilin *** | **2192S** | **Cell Signaling Technology, Danvers, MA** | **Rabbit** | **1:1000 (WB)** |
| **IL-1α *** | **ab7632** | **Abeam, Cambridge, MA** | **Rabbit** | **1:1000 (WB)** |
| **IL-Iβ *** | **ab2105** | **Abcam, Cambridge, MA** | **Rabbit** | **1:1000 (WB)** |
| **IL1-R1*** | **sc-689** | **Santa Cruz Biotechnology, Dallas, Texas** | **Rabbit** | **1:1000 (WB), 1:100 (IHC, ICC)** |
| **β-actin *** | **A5441** | **Sigma Chemical Co., St. Louis, MO** | **Mouse** | **1:10,000 (WB)** |
| **Microtubule associated Protein - 2 (MAP-2) *** | **4542S** | **Cell Signaling Technology, Danvers, MA** | **Rabbit** | **1:1000 (WB), 1:100 (IHC)** |
| **Glial Fibrillary Acidic Protein (GFAP) *** | **SAB4300647** | **Sigma Chemical Co., St. Louis, MO** | **Rabbit** | **1:1000 (WB), 1:100 (IHC)** |
| **RAGE** | **Sc-365154** | **Santa Cruz Biotechnology, Dallas, Texas** | **Mouse** | **1:1000 (WB)** |
| **p-Glycoprotein** | **517310** | **Calbiochem, Millipore, (Temecula, CA).** | **Mouse** | **1:1000 (WB)** |
| **HRP anti-mouse IgG**** | **A9044** | **Sigma Chemical Co., St. Louis, MO** | **Mouse** | **1:2000 (WB)** |
| **HRP anti-rabbit IgG**** | **A0545** | **Sigma Chemical Co., St. Louis, MO** | **Rabbit** | **1:2000 (WB)** |
| **Alexa Fluor 488 anti-mouse IgG**** | **A11001** | **Invitrogen, Carlsbad, CA** | **Mouse** | **1:200 (IHC, ICC)** |
| **Alexa Fluor 546 anti-rabbit IgG**** | **A11010** | **Invitrogen, Carlsbad, CA** | **Rabbit** | **1:200 (IHC, ICC)** |

The following examples are given by way of illustration of the present invention and therefore should not be construed to limit the scope of the present invention.

### Example 1: Treatment of As, Cd and Pb for the development of early wistar rat model of AD

All animal-handling procedures were carried out in accordance with the current regulations of the Indian Institute of Toxicology Research Animal Ethics Committee, and with its prior approval for using the animals. The pregnant female wistar rats were housed in a 12-h day and light cycle environment with ad libitum diet and water. The rats were divided into six groups (n=30 per group), gavage-treated with the metal mixture **(Table 1)**. The metal mixture treatment was at two concentrations **(1X and 10X; Groups 1-3, Table 1)** for dose-dependent study. To identify the synergistic nature, the animals were treated in mixture as well as individually with the metals **(Group 1-6 of Table 1)**. The heavy metals were As, Cd and Pb, fed in the form of NaAsO₂, CdCl₂ and Pb(C₂H₃O₂)₂, respectively, in Milli Q water. The NaAsO₂, and CdCl₂ readily dissolved in water, while Pb(C₂H₃O₂)₂ was dissolved by adding 0.043 % acetic acid in water. The dams (pregnant rats and lactating mother rats) were daily treated with the metals in water from gestation day 5 (G-05) until the pups weaned (postnatal day-21, P-21). From P-22, the postnatal rats were directly treated with the metals until P-90. The tissues were collected at P-24, P-60 or P-90. The male rats were selected for the study, and rats from separate litters served as independent subjects.

In another set, the rats were treated with 10X doses of the metal mixture from P-90 until P-120.

**Table 1: Metal treatment given to pregnant, lactating and post-weaning rats**

| | |
|---|---|
| Group 1, Vehicle | Water (vehicle) |
| Group 2, MM (1X) | NaAsO₂: 0.380 mg/Kg+ CdCl₂: 0.098 mg/Kg+ Pb(C₂H₃O₂)₂: 0.220 mg/Kg |
| Group 3, MM (10X) | NaAsO₂: 3.80 mg/Kg+ CdCl₂: 0.98 mg/Kg+ Pb(C₂H₃O₂)₂: 2.220 mg/Kg |
| Group 4, As-individual treatment | NaASO₂: 11.4 mg/Kg(3x3.80) |
| Group 5, Cd-individual treatment | CdCl₂: 2.94 mg/Kg (3x0.98) |
| Group 6, Pb-individual treatment | Pb(C₂H₃O₂)₂ : 6.660 mg/Kg(3x2.220) |

### Drug treatments:

The known AD-targeting drugs, memantine (10 mg/kg body weight) and donepezil (conc. 1.5 mg/kg body weight) were administered for 10 days along with 10X-MM in P-90 rats. Omega -3 (ω3) fatty acid (mixture of eicosapentaenoic acid, 90mg/Kg, and docosahexaenoic acid, 60mg/Kg) was orally treated from G-0 to P-60 along with 10X-MM (treated from G-05 to P-60). α-tocopherol (100mg/Kg body weight) was orally treated from P-24 to P-60 along with 10X-MM (treated from G-05 to P-60).

### Example 2: Determination of the levels of APP, Aβ, APP-CTF-β, presenilin, IL-1α, IL-1β, IL-1R1, RAGE and pGP in the hippocampus and cortex through western blotting

Tissues of the cerebral cortex and hippocampus from five to seven postnatal wistar rats were harvested, snap-frozen in liquid nitrogen, and stored at -80° C until further investigation. SDS-PAGE and western blotting was performed with APP (1:1000), Aβ-40 (1:1000), Aβ-42 (1:1000), APP-CTF-β (1:1000), presenilin (1:1000), IL-1α (1:1000), IL-1β (1:1000) and IL-1R1 (1:1000), RAGE (1:1000) and pGP (1:1000). The working dilutions for secondary anti-rabbit IgG and anti-mouse IgG conjugated to horseradish peroxidase were 1:2000 in 0.2% Triton X-100 containing PBS. The samples were detected by chemiluminescence with super signal west femto max substrate. Relative expression of each protein was determined by densitometric quantification of blots using VersaDoc Gel Imaging System (BioRad, Hercules, CF).

### Example 3: Determination of the expression of APP, Aβ and Iba-1 in the hippocampus and cortex through immunostaining

Four wistar rat pups from four separate litters, treated with the metal mixture or vehicle, were anaesthetized and perfused, and the cerebral cortex and hippocampus were fixed and cryoprotected. Briefly, five to 10-µM cryostat sections were made using cryomicrotome (Microm HM 520, Labcon, Germany), and mounted on (3-Aminopropyl) triethoxy-silane coated slides. For immunofluorescence, a standard immunofluorescence technique was used. The sections were blocked in 10% normal donkey serum/0.1M PBS and then incubated with the antibodies (1:100) at 4°C for overnight. For detecting the expression of APP,Aβ or Iba-1, the sections were immunostained with monoclonal APP, Aβ or Iba-1antibodies. Following a rinse in 0.1M PBS, the sections were incubated with Alexa Fluor 546 goat anti-rabbit IgG conjugate (1:200) and Alexa Fluor 488 goat anti-mouse IgG conjugate (1:200) for 60 min. After re-rinsing, the sections were counterstained with hoechst 33258 (0.2 mM) for 5 min, cover-slipped on vectashield medium and visualized under a fluorescence microscope. The images were then imported into Image-J 1.42q (http://rsb.info.nih.gov/ ij/; developed by Wayne Rasband, National Institutes of Health, Bethesda, MD) for quantifying cell fluorescence.

### DAB Staining:

For DAB staining, a standard technique was used as described in DAB chromogen and ABC kit. The cryo-sections were blocked in 10% normal donkey serum/0.1M PBS and then incubated with the antibodies, APP (1:100) and Aβ₁₋₄₂ (1:100) at 4°C overnight. Following a rinse in 0.1M PBS, the sections were incubated with secondary antibody for 60 min, and then incubated with ABC reagent for 30 min. After rinsing, the sections were stained with DAB chromogen for 2-5 min, cover-slipped on DPX mounting medium and visualized under optical microscope (Nikon Instech Co. Ltd).

### Example 4: Determination of the BACE enzymatic activity in the cortex and hippocampus

BACE levels in the cortex and hippocampus of wistar rats were detected spectrofluorometrically using the BACE enzyme activity assay. The tissue lysates (50 µl) were added to each well of a black 96-well microplate. Fifty µl of the 2X-Secretase reaction buffer was added to the lysates, followed by the addition of 2 µl of the respective substrate provided in the kits. The plate was covered, tapped gently, kept at 37°C for 1-2 hrs, and then read in a fluorescent microplates reader using excitation (335-355 nm) and emission (495-510 nm) filters.

### Example 5: Determination of the Aβ1-40 through ELISA

The effect of metal mixture treatment on Aβ1-40 levels was determined using specific ELISAs (IBL, Immuno-Biological Co., Ltd., Japan). Cortex and hippocampi from the wistar rats were micro-dissected and quickly homogenized in extraction buffer. The homogenates were diluted 1:10 with cold casein buffer (0.25% casein and 0.05% sodium azide), followed by centrifugation for 30 min at 4°C at 40,000 rpm. Hundred µl of sample was added into the pre-coated plates and incubated overnight at 4°C. After washing each well of the pre-coated plate with washing buffer, 100 µl of labelled antibody solution was added and the mixture was incubated for 1 hr at 4°C in the dark. After washing, the chromogen was added and the mixture was incubated for 30 min at room temperature in the dark. After the addition of stop solution, the resulting colour was assayed at 450 nm using a microplate absorbance reader.

### Example 6: Aβ₁₋₄₂ and IL1-Ra treatment

Recombinant Aβ₁₋₄₂ and recombinant IL1-Ra were injected into rat hippocampus using stereotaxic technique. Briefly, rats were anesthetized with intraperitoneal injection of ketamine and xylazine (60 and 20 mg/kg body weight respectively). The rats were placed in the stereotaxic frames (Stoelting Co., USA), skull exposed and disinfected with betadine. An incision was made into the scalp and drilled (depth of 2.5 mm) at 2.0 mm medial-lateral and 3.5 mm posterior-anterior to bregma. Five µl solution of IL1-Ra (350 ng/ml) or Aβ₁₋₄₂ (1µg/ml) was bilaterally injected slowly into the hippocampus with a 10 µl Hamilton syringe, and syringe retained for 2 minutes for complete diffusion of IL1-Ra or Aβ₁₋₄₂. IL1-Ra was injected, once, at P-90 in 10X-MM-treated rats, and dissected after 10 days. Aβ₁₋₄₂ was injected, once, in P-90 control male rats and dissected after 10 days. The needle was slowly withdrawn after injection. Control group, treated with sterile PBS, underwent the same procedures.

### Example 7: Determination of Combination index

To characterize the synergistic interaction between the heavy metals for their effects on the APP, Aβ and BACE levels, a combination index (CI) was calculated using the software Calcusyn (Biosoft, Manchester, United Kingdom). CI values <1.0 indicated synergism (Zhao et al., 2004).

### Example 8: Detection of Aβ-mediated apoptosis (in vivo) in the neurons and astrocytes of rat cortex and hippocampus through TUNEL assay (with Aβ-42)

Detection of apoptosis (*in vivo*) in MAP-2 expressing neurons and GFAP-ir astrocytes was performed. In situ detection of apoptosis was carried out by TUNEL assay. Briefly, four pups from four different litters were taken at the developmental stages, anesthetized, and perfused and the brain was fixed and cryoprotected. Five-micron sections from the cortex and hippocampus were made using cryomicrotome (Microm HM 520; Labcon). For the TUNEL assay, a labelling reaction was carried out with fluorescein-labelled dUTP in the presence of TdT at 37°C for 1 h. To investigate whether the apoptotic cells were neurons or astrocytes, the sections were immunostained with anti-mouse MAP-2 or GFAP antibody (1:100 dilution in TBST [10mM Tris, pH 8.0, 150mM NaCl, 0.01% Tween 20]) according to manufacturer's protocol. The sections were then incubated with Alexa Fluor 546-conjugated (fluorescent color: red; Abs/Em: 555/565) goat anti-mouse antibody (1:200 dilution); counterstained with Hoechst 33258 (0.2mM) for 5 min; and visualized under a fluorescence microscope (Nikon Instech Co. Ltd) after being coverslipped on Vectashield medium (Vector Laboratories). For Aβ immunofluorescence, a standard immunofluorescence technique was used in serial sections of that used for GFAP/MAP-2 and TUNEL assay. The sections were blocked in 10% normal donkey serum/0.1M PBS and then incubated with the antibodies (1:100) at 4°C for overnight. Following a rinse in 0.1M PBS, the sections were incubated with Alexa Fluor 546 goat anti-mouse IgG conjugate (1:200). After re-rinsing, the sections were counterstained with hoechst 33258 (0.2 mM) for 5 min, cover-slipped on vectashield medium and visualized under a fluorescence microscope. The images were then imported into Image-J 1.42q (http://rsb.info.nih.gov/ ij/; developed by Wayne Rasband, National Institutes of Health, Bethesda, MD) for quantifying cell fluorescence.

### Example 9: Passive avoidance test

The rats were subjected to the passive avoidance test by placing in a compartment of computerized shuttle box (Techno, India). The light compartment was isolated from the dark compartment by an automated guillotine door. After an acclimatization period of 30 s, the guillotine door was opened and closed automatically after entry of the rat into the dark compartment. The subject received a low-intensity foot shock (0.5mA; 10 s) in the dark compartment. Infrared sensors monitored the transfer of the animal from one compartment to another, which was recorded as transfer latency time (TLT) in seconds. The 1^{st} trial was for acquisition and retention was tested in a 2^{nd} trial (1^{st} retention) given 24 h after the 1^{st} trial. The duration of a trial was 300s. Further, 2^{nd}, 3^{rd} and 4^{th} retention trials were given on alternate days to test retention in the metal mixture treated rats. The shock was not delivered in the retention trials to avoid reacquisition. The criterion for learning was taken as an increase in the TLT on retention (2^{nd} or subsequent) trials as compared to acquisition (1^{st}) trial.

### Example 10: Primary neuronal culture

The pregnant wistar rats were sacrificed by cervical dislocation and the embryos were removed on the 16^{th} day of gestation. The embryonic brain tissues were mechanically dissociated into individual cells in dissection media containing Glucose (1M), sucrose (1 M), HEPES Buffer (1M) and Hank's salt (1X). The resulting cells were centrifuged (1,500 rpm, 5 min), resuspended in NEUROBASAL medium containing B-27 supplement (Invitrogen, Carlsbad, CA), L-glutamine (0.5 mM), penicillin (100 U/ml) and streptomycin (100µg/ml) and plated into 60 mm dishes. The culture media was changed every 2 days. Greater than 90% of the cells in these cultures were neurons as assessed by cell morphology and immunostaining with rabbit monoclonal antibodies against MAP-2 (1: 100).

### Example 11: Primary astrocyte culture

The astrocytes from rat brain were isolated and cultured. Briefly, prefrontal cortices of 1-day-old wistar rats were dissected out and digested with trypsin for 10 min at 37°C. A single cell suspension was obtained by triturating, and the cells were seeded onto poly-L-lysine (100 µg/ml)-coated plates. The cultures were maintained in DMEM/F12 with 10% heat-inactivated fetal bovine serum, 100 U/ml penicillin, and 100 mg/ml streptomycin. The confluent cells were rinsed twice with serum-free media and then detached with 0.25% trypsin with ethylenediaminetetraacetic acid and sub-cultured. The cells reached confluence at 7 days after subculture, and at this point, more than 95-97% of cells were GFAP-positive astrocytes, as determined by the immunofluorescence staining.

### Example 12: Cell treatment

The astrocytes and neurons were grown to 80% confluence, pre-incubated in reduced serum (0.5% fetal bovine serum [FBS]) medium for 2 h, and then treated with a mixture of As, Cd, and Pb **(Group 1 and 2, Table 2 for neurons, and Table 3 for astrocytes)** in the form of NaAsO₂, CdCl₂ and Pb(C₂H₃O₂)₂, respectively in Milli Q water for 16 h, and incubated in a humidified tissue culture incubator at 37°C with 5% CO2-95% air. The NaAsO₂, and CdCl₂ readily dissolved in water, while Pb(C₂H₃O₂)₂ was dissolved by adding 0.043 % acetic acid in water. To identify the synergistic nature, the astrocytes and neurons were treated with metal mixture as well as individual metals **(Group 1-5 of Table 2 for neurons, and Table 3 for astrocytes).**

**Table 2: Metal treatment given to neurons**

| | |
|---|---|
| Group 1: Vehicle | Water (vehicle) |
| Group 2: metal mixture (MM) | NaAsO₂: 5 (µM + CdCl₂: 1 µM + Pb(C₂H₃O₂)₂: 10 µM |
| Group 3: As-individual treatment | NaAsO₂: 15 µM (3x5 µM) |
| Group 4: Cd-individual treatment | NaAsO₂: 3 µM (3x1 µM) |
| Group 5: Pb-individual treatment | Pb(C₂H₃O₂)₂: 30 µM (3x10 µM) |

**Table 3: Metal treatment given to astrocytes**

| | |
|---|---|
| Group 1: Vehicle | Water (vehicle) |
| Group 2: MM | NaAsO₂: 6 µM + CdCl₂: 2 µM + Pb(C₂H₃O₂)₂: 50 µM |
| Group 3: As-individual treatment | NaAsO₂: 18 µM (3x6 µM) |
| Group 4: Cd-individual treatment | NaAsO₂: 6 µM (3x2 µM) |
| Group 5: Pb-individual treatment | Pb(C₂H₃O₂)₂: 150 µM (3x50 µM) |

### DHA and α-tocopherol treatment:

The astrocytes and neurons were grown to 80% confluence, pre-incubated in reduced serum (0.5% fetal bovine serum [FBS]) medium for 2 h, and then treated with a mixture of As, Cd, and Pb with DHA(10 µM) or α-tocopherol (10 µM) for 16 h, and incubated at 37°C.

### Example 13: Determination of the levels of APP, Aβ, IL-1α, IL-1β and IL-1R1 in the astrocytes and neurons

The astrocytes and neurons were treated, washed with PBS and suspended in 100 µl of CelLytic™ MT Cell Lysis Reagent protease inhibitor cocktail (a mixture of 4-(2-aminoethyl) benzenesulfonyl fluoride, pepstatinA, E-64, bestatin, leupeptin, and aprotinin)] and kept on ice for 20 min. The cells were homogenized using a Teflon homogenizer and centrifuged at 15, 000 rpm for 30 min at 4°C, and the supernatant was collected. SDS-PAGE and western blotting was done with APP (1:1000), Aβ-40 (1:1000), Aβ-42 (1:1000), IL-1 α(1:1000), IL-1 β (1:1000) and IL-1R1. The working dilutions for secondary anti-rabbit IgG and anti-mouse IgG conjugated to horseradish peroxidase were 1:2000 in 0.2% Triton X-100 containing PBS. The samples were detected by chemiluminescence with super signal west femto max substrate. Relative expression of each protein was determined by densitometric quantification of blots using VersaDoc Gel Imaging System (BioRad, Hercules, CF).

### Example 14: Immunocytochemistry

The astrocytes and neurons at 80 % confluence were fixed with 4% PFA for 1 hr at room temperature, followed by three rinses in PBS. Cells were then pre-incubated for 15-30 min with PBS containing 0.3% Triton X-100 (Sigma) and 3% normal horse serum (Gibco-BRL) at room temperature. Cultures were then incubated overnight with antibodies directed against APP (1:100), Aβ-40 (1:100), Aβ-42 (1:100), IL-1α (1:100), lL-1β (1:100) and IL-1R1 (1:100) and diluted in PBS containing 0.3% Triton X-100 and 5% normal horse serum. Following a rinse in 0.1M PBS, the sections were incubated with Alexa Fluor 546 goat anti-mouse IgG conjugate (1:200) for 60 min. After re-rinsing, the sections were counterstained with hoechst 33258 (0.2 mM) for 5 min, cover-slipped on vectashield medium and visualized under a fluorescence microscope. The images were then imported into Image-J 1.42q (http://rsb.info.nih.gov/ ij/; developed by Wayne Rasband, National Institutes of Health, Bethesda, MD) for quantifying cell fluorescence.

### Example 15: BACE activity in cells

The BACE activity in the cell lysates was detected spectrofluorometrically using BACE enzyme activity assay. The cell lysate, in buffer provided with the kit (50 µl) were added to each well of a black 96-well microplate. The protocol is the same as that for tissue BACE assay.

### Observed properties

### Animal (rat model)

Upon treating the developing wistar rats with the metal mixture from G-05, the rats demonstrated a dose- dependent increase in the Aβ-petides and APP levels in the cortex **(Table 4)** and hippocampus **(Table 5)**. The effect was synergistic, with a CI value less than 1.0. The known AD-targeting drugs, memantine and donepezil suppressed the levels of Aβ and APP in this non-transgenic wistar rat model of AD **(Table 6)**. The metal mixture induced an increase in BACE activity in the rat cortex and hippocampus, at an early age **(Table 7).** The effect was synergistic, with a CI value less than 1.0. The metal mixture induced APP-CTF-β and presenilin at an early age **(Table 8).** The metal mixture induced Aβ-mediated apoptosis in the rat brain **(Table 9)**. The metal mixture induced a loss in cognition at an early age **(Table 10).** The metal mixture induced the inflammatory cytokines IL-1α, IL-1β and their receptor, IL-1R1, that contribute towards AD pathogenesis **(Table 11)**. Upon treating the developing wistar rats with the metal mixture from P-90 to P-120, the rats demonstrated an increase in the Aβ-peptides and APP levels **(Table 12)**.

### Animal data

**Table 4: Dose-dependent increase in Aβ-42, Aβ-40 and APP in rat brain cortex upon exposure to the metal mixture**

| **Aβ-42 (fold increase compared to age- matched vehicle)** | | **Aβ-40 (fold increase compared to age- matched vehicle)** | | **APP (fold increase compared to age- matched vehicle)** | |
|---|---|---|---|---|---|
| 1X | 10X | 1X | 10X | 1X | 10X |
| 1.7 ± 0.1028 | 2.4 ± 0.1098 | 1.3 ± 0.1063 | 1.5 ± 0.1052 | 1.5 ± 0.0959 | 2.2 ± 0.1032 |

**Table 5: Dose-dependent increase in Aβ-42, Aβ-40 and APP in rat brain hippocampus upon exposure to the metal mixture**

| **Aβ-42 (fold increase compared to age- matched vehicle)** | | **Aβ-40 (fold increase compared to age- matched vehicle)** | | **APP (fold increase compared to age- matched vehicle)** | |
|---|---|---|---|---|---|
| 1x | 10X | 1X | 10X | 1X | 10X |
| 1.7 ± 0.0875 | 3.0 ± 0.1452 | 1.5 ± 0.1077 | 1.8 ± 0.1909 | 1.3 ± 0.0461 | 3.0 ± 0.1498 |

**Table 6: Reduction in the metal mixture-induced Aβ by memantine and donepezil (two week treatment) in rat brain**

| **Compounds** | **fold reduction in Aβ-40** | **fold reduction in Aβ-42** | **fold reduction in APP** |
|---|---|---|---|
| **Memantine** | 2.54 ± 0.0099 | 2.42. ± 0.0143 | 1.69 ± 0.0510 |
| **Donepezil** | 3.5 ± 0.0799 | 3.22 ± 0.0800 | 3.14 ± 0.2160 |

**Table 7: Increase in BACE activity in rat brain upon exposure to the metal mixture**

| **Rat age** | **BACE activity-Cortex (fold increase compared to age- matched vehicle)** | **BACE activity-hippocampus (fold increase compared to age- matched vehicle)** |
|---|---|---|
| **Postnatal 60 d** | 1.3 ± 0.1450 | 1.26 ± 0.0510 |
| **Postnatal 90 d** | 2.0 ± 0.2679 | 1.5 ± 0.2733 |

**Table 8: Increase in APP-CTF-β and presenilin in rat brain upon exposure to the metal mixture**

| | **Fold increase compared to age- matched vehicle in cortex** | **Fold increase compared to age- matched vehicle in hippocampus** |
|---|---|---|
| **APP-CTF-β** | 1.3 ± 0.0908 | 1.5 ± 0.1347 |
| **Presenilin** | 1.7 ± 0.1635 | 1.6 ± 0.1066 |

**Table 9: Increase in Aβ-mediated apoptosis in rat brain upon exposure to the metal mixture**

| **Rat age** | **Apoptosis-brain (fold increase compared to age-matched vehicle)** |
|---|---|
| **Postnatal 60 day** | 11.0 ± 2.1369 |
| **Postnatal 90 day** | 16.0 ± 1.3558 |

**Table 10: Loss in cognition upon exposure to the metal mixture**

| **Rat age** | **Loss in cognition (approx.)** |
|---|---|
| **Postnatal 90 d** | 35 % |

**Table 11: Increase in the levels of inflammatory markers in the rat brain upon exposure to the metal mixture**

| **Rat age** | **IL-1α (fold increase compared to age-matched vehicle)** | **IL-1β (fold increase compared to age-matched vehicle)** | **IL-1R1 fold increase compared to age-matched vehicle)** |
|---|---|---|---|
| **Postnatal 60 day** | 1.6 ± 0.1794 | 1.3 ± 0.1194 | 1.3 ± 0.1104 |
| **Postnatal 90 day** | 1.7 ± 0.1037 | 1.8 ± 0.1901 | 1.5 ± 0.0769 |

**Table 12: Increase in Aβ-42 and APP in P-120 rat brain upon exposure to the metal mixture from P-90**

| **Aβ-42 (fold increase compared to age- matched vehicle)** | **APP (fold increase compared to age- matched vehicle)** |
|---|---|
| 1.5 ± 0.2274 | 1.4 ± 0.0808 |

### Example 16: Cellular (neuronal and astrocyte models)

Upon treating the neuronal and astrocyte cells with the metal mixture, the cells demonstrated an increase in the Aβ-peptides and APP levels, and BACE activity **(Table 13)**. The effect was synergistic, with a CI value less than 1.0. The known AD-targeting drugs, memantine and donepezil suppressed the levels of Aβ and APP in the non-transgenic neuronal and astrocyte models of AD. The omega-3 fatty acid (docosahexaenoic acid), known to reduce the risk of AD, suppressed the levels of Aβ and APP in the non-transgenic neuronal and astrocyte models of AD. Vitamin E, known to be protective against AD, suppressed the levels of Aβ and APP in the non-transgenic neuronal and astrocyte models of AD **(Table 14)**.

The metal mixture induced the inflammatory cytokines IL-1α and IL-1βin the brain cells **(Table 15)**.

### In vitro data

**Table 13: Increase in Aβ, APP and BACE in neurons and astrocytes upon exposure to the metal mixture**

| **Cell type** | **Aβ-fold increase** | **APP-fold increase** | **BACE-fold increase** |
|---|---|---|---|
| Neuron | 2.3 ± 0.2232 | 2.0 ± 0.1949 | 1.5 ± 0.1917 |
| Astrocyte | 2.2 ± 0.2015 | 2.3 ± 0.2247 | 2.0 ± 0.2682 |

**Table 14: Reduction in metal mixture-induced Aβ and APP in rat astrocytes and neurons by memantine, donepezil, docosahexaenoic acid and Vit. E**

| **Compounds** | **Fold reduction in Aβ** | **Fold reduction in APP** |
|---|---|---|
| Memantine | 1.4 ± 0.052 | 1.3 ± 0.0392 |
| Donepezil | 1.8 ± 0.038 | 2.0 ± 0.0384 |
| Docosahexaenoic acid | 2.3 ± 0.0853 | 1.25 ± 0.0265 |
| α-tocopherol | 1.9 ± 0.0192 | 1.3 ± 0.0493 |

**Table 15: Increase in the levels of inflammatory markers in the rat brain cells exposure to the metal mixture**

| **IL-1α (fold increase -matched vehicle)** | **IL-1β (fold increase compared to vehicle)** |
|---|---|
| 1.7 ± 0.0600 | 1.5 ± 0.1021 |

### Advantages

This method of invention of the non-transgenic early wistar rat model of AD is very novel, with respect to inducing early signs of AD synergistically. Its advantages over the known methods are,
(a) reduced mechanical tissue damage from intra-cranial administration procedures,
(b) non-neurotoxicity of vehicle, and
(c) convenient method of induction. Its advantages over the production of transgenic animals are (a) less laborious protocol and (b) the ethical concerns that limit the numbers of animals employed in experiments.

The method is more beneficial compared to the transgenic lower species that have a brain anatomy much different from humans.

This non-transgenic model shows a range of symptoms, such as the generation of the pathological Aβ-40, 42 peptides, the APP, Aβ-mediated apoptosis and vascular damage along with a rise in the inflammatory markers that are reported to aggravate the disease pathogenicity. The time needed to generate the models is less, and the method is less expensive.

Moreover, our non-transgenic animal model is validated with the known AD-targeting drugs and preventive agents.

The model does not show toxicity of the other vital organs.

This method of invention of the non-transgenic cellular model of AD is very novel, with respect to inducing signs of AD in neurons and astrocytes, and synergistically.

The non transgenic *in vitro* model for AD is less expensive, quick and efficient.

It is validated with the known AD-targeting drugs and preventive agents.

Moreover, treatment with the metal mixture also serves as a model-inducer for cells already over-expressing the amyloidogenic APP.

## Claims

1. Use of
a non-transgenic animal wistar rat model of early Alzheimer's disease **characterized in** having over-expression of Amyloid Beta (Aβ), amyloid precursor protein (APP), β-secretase (BACE), C-terminal fragment beta (CTF-β) and presenilin proteins in the rat brain cortex and hippocampus, induced by exposing the rat from postnatal day 90 until postnatal day 120 to a mixture of heavy metals comprising arsenic, cadmium and lead in the form of NaAsO₂, CdCl₂ and Pb(C₂H₃O₂)₂ at 3.8 mg/Kg, 0.98 mg/Kg, 2.20 mg/Kg respectively, in water,
for screening anti-Alzheimer's drug.

2. Use of the rat model as claimed in claim 1, for developing anti- Alzheimer's therapies.

3. Use of the rat model as claimed in claim 1, for detecting early age Alzheimer's disease.

4. Use of the rat model as claimed in claim 1 for screening drugs and developing therapies targeted to BACE, CTFβ and presenilin.

5. Use of a non-transgenic wistar rat cellular neuronal model of Alzheimer's disease **characterized in** having over-expression of Amyloid Beta (Aβ), amyloid precursor protein (APP), β-secretase(BACE) and presenilin proteins by exposing neuronal cells of wistar rats to a mixture of heavy metals arsenic, cadmium and lead for screening anti-Alzheimer's drugs.

6. Use of the rat model as claimed in claim 5, for developing anti- Alzheimer's therapies.

7. Use ofa non-transgenic wistar rat cellular astrocyte model of Alzheimer's disease having over-expression of Amyloid Beta (Aβ), amyloid precursor protein (APP), β-secretase (BACE) and presenilin proteins by exposing astrocyte cells of wistar rats to a mixture of heavy metals arsenic, cadmium and lead for screening anti- Alzheimer drugs.

8. Use of the non-transgenic wisar rat astrocyte model as claimed in claim 7 for developing anti-Alzheimer therapies.

## Patentansprüche

1. Verwendung
eines nicht-transgenen Wistarratten-Tiermodells von frühem Morbus Alzheimer, welches durch das Aufweisen von Überexpression von Amyloid Beta (Aβ), Amyloid-Vorläuferprotein (APP), β-Sekretase (BACE), C-terminalem Fragment beta (CTF-β) und Presenilin-Proteinen im Rattenhirn-Cortex und -Hippocampus gekennzeichnet ist, induziert durch Einwirkenlassen auf die Ratte vom Tag 90 nach der Geburt bis zum Tag 120 nach der Geburt von einem Gemisch von Schwermetallen, umfassend Arsen, Cadmium und Blei in der Form von NaAsO₂, CdCl₂ und Pb(C₂H₃O₂) zu 3,8 mg/kg, 0,98 mg/kg beziehungsweise 2,20 mg/kg in Wasser,
zum Screenen eines Anti-Alzheimer-Arzneistoffes.

2. Verwendung des Rattenmodells wie in Anspruch 1 beansprucht zum Entwickeln von Anti-Alzheimer-Therapien.

3. Verwendung des Rattenmodells wie in Anspruch 1 beansprucht zum Nachweisen von früheinsetzendem Morbus Alzheimer.

4. Verwendung des Rattenmodells wie in Anspruch 1 beansprucht zum Screenen von Arzneistoffen und Entwickeln von Therapien, welche auf BACE, CTFβ und Presenilin zielgerichtet sind.

5. Verwendung eines nicht-transgenen neuronalen Wistarratten-Zellmodells von Morbus Alzheimer, welches durch das Aufweisen von Überexpression von Amyloid Beta (Aβ), Amyloid-Vorläuferprotein (APP), β-Sekretase (BACE) und Presenilin-Proteinen **gekennzeichnet ist, durch** Einwirkenlassen auf neuronale Zellen von Wistarraten von einem Gemisch der Schwermetalle Arsen, Cadmium und Blei
zum Screenen von Anti-Alzheimer-Arzneistoffen.

6. Verwendung des Rattenmodells wie in Anspruch 5 beansprucht zum Entwickeln von Anti-Alzheimer-Therapien.

7. Verwendung eines nicht-transgenen Wistarratten-Astrozytenzellmodells von Morbus Alzheimer mit Überexpression von Amyloid Beta (Aβ), Amyloid-Vorläuferprotein (APP), β-Sekretase (BACE) und Presenilin-Proteinen durch Einwirkenlassen auf Astrozytenzellen von Wistarratten von einem Gemisch der Schwermetalle Arsen, Cadmium und Blei
zum Screenen von Anti-Alzheimer-Arzneistoffen.

8. Verwendung des nicht-transgenen Wistarratten-Astrozytenmodells wie in Anspruch 7 beansprucht zum Entwickeln von Anti-Alzheimer-Therapien.

## Revendications

1. Utilisation d'un modèle animal de rat wistar non transgénique de maladie d'Alzheimer précoce **caractérisé en ce qu'**il présente une surexpression d'amyloïde bêta (Aβ), de la protéine précurseur d'amyloïde (APP), de β-sécrétase (BACE), du fragment bêta C-terminal (CTF-β) et de protéines préséniline dans le cortex cérébral et l'hippocampe du rat, induite par l'exposition du rat à partir du jour postnatal 90 jusqu'au jour postnatal 120 à un mélange de métaux lourds comprenant de l'arsenic, du cadmium et du plomb sous la forme de NaAsO₂, CdCl₂ et Pb(C₂H₃O₂)₂ à 3,8 mg/kg, 0,98 mg/kg, 2,20 mg/kg respectivement, dans de l'eau,
pour le criblage d'un médicament anti-Alzheimer.

2. Utilisation du modèle de rat selon la revendication 1, pour le développement de thérapies anti-Alzheimer.

3. Utilisation du modèle de rat selon la revendication 1, pour détecter une maladie d'Alzheimer à un âge précoce.

4. Utilisation du modèle de rat selon la revendication 1 pour cribler des médicaments et développer des thérapies ciblant la BACE, la CTF-β et la préséniline.

5. Utilisation d'un modèle neuronal cellulaire de rat wistar non transgénique de maladie d'Alzheimer **caractérisé en ce qu'**il présente une surexpression d'amyloïde bêta (Aβ), de la protéine précurseur d'amyloïde (APP), de β-sécrétase (BACE) et de protéines préséniline par l'exposition de cellules neuronales de rats wistar à un mélange des métaux lourds arsenic, cadmium et plomb
pour le criblage de médicaments anti-Alzheimer.

6. Utilisation du modèle de rat selon la revendication 5, pour le développement de thérapies anti-Alzheimer.

7. Utilisation d'un modèle d'astrocyte cellulaire de rat wistar non transgénique de maladie d'Alzheimer présentant une surexpression d'amyloïde bêta (Aβ), de la protéine précurseur d'amyloïde (APP), de β-sécrétase (BACE) et de protéines préséniline par l'exposition de cellules d'astrocyte de rats wistar à un mélange des métaux lourds arsenic, cadmium et plomb
pour le criblage de médicaments anti-Alzheimer.

8. Utilisation du modèle d'astrocyte de rat wistar non transgénique selon la revendication 7, pour le développement de thérapies anti-Alzheimer.
